Europäisches Patentamt

European Patent Office

Office européen des brevets

(11) Publication number: **0 365 525 B1**

## EUROPEAN PATENT SPECIFICATION

(45) Date of publication of patent specification: **09.08.95**

(51) Int. Cl.⁶: **C07K 7/06**, C07K 7/08, C07K 14/00, A61K 38/00, C12Q 1/04

(21) Application number: **88901989.9**

(22) Date of filing: **27.01.88**

(86) International application number:
**PCT/US88/00245**

(87) International publication number:
**WO 88/05784 (11.08.88 88/18)**

(54) **LYMPHOCYTE INHIBITION BY HLA PEPTIDES.**

(30) Priority: **30.01.87 US 8846**
    **24.12.87 US 138547**

(43) Date of publication of application:
**02.05.90 Bulletin 90/18**

(45) Publication of the grant of the patent:
**09.08.95 Bulletin 95/32**

(84) Designated Contracting States:
**AT BE CH DE FR GB IT LI LU NL SE**

(56) References cited:

**The Journal of Immunology, Volume 134, issued April, 1985 (KOLLER et al), "Cloning and Complete Sequence of an HLA-A2 Gene: Analysis of Two HLA-A Alleles at the Nucleotide Level" pages 2727-2733. see page 2731 in particular.**

(73) Proprietor: **THE BOARD OF TRUSTEES OF THE LELAND STANFORD JUNIOR UNIVERSITY**
**Encina 105**
**Stanford University**
**Stanford**
**California 94305 (US)**

(72) Inventor: **KRENSKY, Alan, M.**
**812 Mayfield Avenue**
**Stanford, CA 94305 (US)**
Inventor: **PARHAM, Peter**
**734 Alvarado Court**
**Stanford, CA 94305 (US)**
Inventor: **CLAYBERGER, Carol**
**812 Mayfield Avenue**
**Stanford, CA 94305 (US)**

(74) Representative: **Goldin, Douglas Michael et al**
**J.A. KEMP & CO.**
**14, South Square**
**Gray's Inn**
**London WC1R 5LX (GB)**

Proc. Natl. Acad. Sci. USA, Volume 82, issued November, 1985 (VEGA et al), "Structural Analysis of an HLA-B27 Functional Variant: Identification of Residues that Contribute to the Specificity of Recognition by Cytolytic T Lymphocytes", pages 7394-7398. see p. 7396, 7397, 7398 in particular.

CHEMICAL ABSTRACTS, (Columbus, Ohio, USA), Volume 106, issued 1987, (PARHAM et al), "Inhibition of Alloreactive Cytotoxic T Lymphocytes by Peptides from the alpha 2 Domain of HLA-A2, see page 516, column 1, the Abstract No. 154384x, Nature, 1987 325 (6105), 625-8, (Eng.).

## Description

FIELD OF THE INVENTION

HLA peptide compositions affect T-cell activity subject to MHC restriction. Methods and compositions are provided involving the modulation of cytotoxicity toward allogeneic cells.

BACKGROUND OF THE INVENTION

Cytotoxic T-cells, particularly cytotoxic T-lymphocytes (CTL), are restricted in their activity by recognizing a specific major histocompatibility (MHC) antigen on the surface of the target cell, as well as an antigen exogenous to the host. The foreign antigen may be as a result of transplantation, viral infection, mutation, or the like. The presence of the determinant or restricting site of the MHC protein appears to be essential to the attack by the CTL against the cell carrying the foreign antigen. In this way, the immune system is able to destroy cells in the body, which, if otherwise allowed to proliferate, would result in the proliferation of pathogens or neoplastic cells.

The CTL's are also effective in destroying transplants of various organs or tissue which come from allogeneic hosts. The methods or treatments employed for protecting the transplanted cells frequently result in the incapacitating of the immune system, subjecting the transplant recipient to opportunistic infection.

It is, therefore, of substantial interest to be able to modulate the cytotoxic system in a host to selectively inhibit a particular population of T-cells from performing their normal physiological function. In this manner, the immune system is substantially maintained intact, while a particular T-cell target may be protected.

DESCRIPTION OF THE RELEVANT LITERATURE

Clayberger et al., J. Exp. Med. (1985) 11:1709-1714 describe HLA-A2 antigen in comparisons with HLA-Aw68 and Aw69. Further structural information concerning human histocompatibility antigens is also available from Vega et al, Proc. Natl. Acad. Sci. USA (1985) 82:7394-7398 who have sequenced an HLA-B27 variant and from de Castro et al, Biochemistry (1983) 22: 3969-3975 who have determined the primary structure of HLA-B40 (-Bw60) and outlined its allogenic sites. Townsend et al.,.Cell, (1986) 44:959-968 suggests that CTL recognize segmental epitopes of denatured or degraded proteins in a similar way as helper T-cells. Holmes and Parham, EMBO J. (1985) 4:2849-2854 describe the relationship of HLA-A2. Aw68 and Aw69. CTL target specificity has been taught to be extremely sensitive to changes in structure of human Class I molecules (Durna and Pease, Transplantation, (1986) 41:279-285: Biddison, et al.,J. Immunol., (1980) 124:548-552: Spits, et al., Immunogenetics, (1982) 16:503-512: Gaston, et al., J. Exp. Med., (1983) 158:280-293).

Mutants which affect recognition by CTL have been studied in mice (Nathenson et al., Ann. Rev. Immunol. (1986) 4:471-502: Schulz et al., Proc. Natl. Acad. Sci. USA (1983, 80:207-2011) and humans, (Krangel Biochemistry (1982) 21:6313-6321: Krangel et al., J. Immunol. (1983) 130:1856-1862: Cowan et al., J. Immunol. (1985) 135:2835-2841: Taketani et al., ibid (1984) 133:816-821; and Vega et al., Proc. Natl. Acad. Sci. USA (1985) 82:7394-7398).

These reports have focused considerable attention on the region between residues 147 and 157, although other regions can also produce functional differences (Ezquerra et al., J. Immunol. (1985) 134:2727 2733). Clusters of variability have been reported at the carboxy-terminal end of the first extracellular domain and at the amino-terminal end of the second extracellular domain (Ways et al., J. Biol. Chem. (1985) 26:11924-11933). Sequences between residues 105-108 of all Class 1 molecules are related to that of the fibronectin binding tetrapeptide (Auffray and Novotny J. Human Immunology (1986) 15:381-390), which tetrapeptide in either orientation is found to have cell attachment properties (Pierschbacher and Ruoslahti, Nature (1984) 309:30-33; Yamada and Kennedy, J. Cell Biol. (1985) 28:99-104). Substitution at position 107 affecting a single monoclonal antibody defined epitope of HLA-A2 has been reported by Salter et al, J. Exp. Med. (1987) 166:283-288.

SUMMARY OF THE INVENTION

Methods and compositions are provided for modulating cytotoxic T-lymphoctye (CTL) activity toward target cells. The methods employ using peptide fragments cross-reactive with the region encompassing the $\alpha_1$- and $\alpha_2$-domains of major histocompatibility Class I antigens. Particularly, fragments which include at least a portion of the amino acids between positions 55 and 120 of the Class I antigen are employed.

Accordingly one aspect of the invention is a peptide linked to a functional moiety selected from a labelling group, an immunogen, a polypeptide antigen, a linker, and a cytotoxic agent;

wherein the peptide modulates CTL (cytotoxic T-lymphocyte) activity and consists of a sequence of a least 8 amino acids coming within the extended sequence

$$\text{aa}^{55} \; G \; P \; E \; Y \; W \; D \; \text{aa}^{62} \; \text{aa}^{63} \; T \; \text{aa}^{65} \; \text{aa}^{66} \; \text{aa}^{67} \; K \; \text{aa}^{69} \; \text{aa}^{70} \; \text{aa}^{71}$$

$$Q \; T \; \text{aa}^{74} \; R \; \text{aa}^{76} \; \text{aa}^{77} \; L \; \text{aa}^{79} \; \text{aa}^{80} \; \text{aa}^{81} \; \text{aa}^{82} \; \text{aa}^{83} \; Y \; Y \; N \; Q \; S \; E \; A \; G$$

$$S \; H \; \text{aa}^{94} \; \text{aa}^{95} \; Q \; \text{aa}^{97} \; M \; \text{aa}^{99} \; G \; C \; D \; \text{aa}^{103} \; G \; \text{aa}^{105} \; D \; \text{aa}^{107} \; R \; \text{aa}^{109} \; L \; R$$

$$G \; \text{aa}^{113} \; \text{aa}^{114} \; Q \; \text{aa}^{116} \; A \; Y \; D \; G$$

wherein:

$\text{aa}^{55}$ is E or K;

$\text{aa}^{62}$ is G, Q, E, or R;

$\text{aa}^{63}$ is an acidic amino acid or amide thereof;

$\text{aa}^{65}$ is Q, R or G;

$\text{aa}^{66}$ is I, N or K;

$\text{aa}^{67}$ is an aliphatic neutral amino acid;

$\text{aa}^{69}$ is an aliphatic neutral amino acid;

$\text{aa}^{70}$ is Q, H, S, N, or K;

$\text{aa}^{71}$ is an aliphatic neutral amino acid;

$\text{aa}^{74}$ is D, Y or H;

$\text{aa}^{76}$ is E or V;

$\text{aa}^{77}$ is D, S or N;

$\text{aa}^{79}$ is R or G;

$\text{aa}^{80}$ is T, I or N;

$\text{aa}^{81}$ is an aliphatic non-polar amino acid;

$\text{aa}^{82}$ is R or L;

$\text{aa}^{83}$ is G or R;

$\text{aa}^{94}$ is T or I;

$\text{aa}^{95}$ is a non-polar aliphatic amino acid of from 5 to 6 carbon atoms;

$\text{aa}^{97}$ is an aliphatic amino acid or W;

$\text{aa}^{99}$ is an aromatic amino acid;

$\text{aa}^{103}$ is a non-polar aliphatic amino acid of from 5 to 6 carbon atoms;

$\text{aa}^{105}$ is P or S;

$\text{aa}^{107}$ is G or W;

$\text{aa}^{109}$ is L or F;

$\text{aa}^{113}$ is Y or H;

$\text{aa}^{114}$ is H, Q, D, N, or R;

$\text{aa}^{116}$ is Y, D, S, F, or H;

with the proviso that the peptide is not a labelled peptide of the formula:

(i) R E T Q I C K A K

(ii) A Q T D R E $\text{aa}^{77}$ L R

where $\text{aa}^{77}$ is D, S or N;

(iii) G Y Y N Q S E A G S H T L Q $\text{aa}^{97}$

where $\text{aa}^{97}$ is S or R;

(iv) Y G C D V G P D G R.

Preferred examples of such peptides are those that consist of at least 8 amino acids coming within the sequence:

G S H T [V, I, or L] Q R M Y G C D V G S D [W or G] R

F L R G Y H Q Y A Y D G.


; or


Q E G P E Y W D (G or R) (E or N) T (R or Q) (K or N)

V K A (H or Q) S Q T  (H or D) R (V or E) (D, S or N) L

(G or R) (T or I) (L or A) (R or L) (G or R) Y Y N Q S E A,


wherein the amino acids in the parentheses indicate that any of one amino acids in the parentheses is present.

Such peptides may be extended at either the N- or C-terminus by an amino acid sequence other than the wild type sequence of the protein from which said peptide is derived.

Another aspect of the invention is a pharmaceutical composition which comprises a peptide as defined above, present in a pharmacologically effective dose in a pharmaceutically acceptable medium.

Preferred such pharmaceutical compositions comprise a peptide or peptides selected from

T L Q R M Y G C D V G S D W R F L R G,

M Y G C D V G S D W R F L R G Y,

M Y G C D V G S D G R F L R G Y,

G P E Y W D G E T R K V K A, and

G P E Y W D R N T R N V K A.

Yet another aspect of the invention is an ex vivo method for determining the presence of MHC restricted cytotoxic T-lymphocytes (CTLs) which comprises contacting cytotoxic T-lymphocytes with a peptide as defined in any one of the above, said peptide covalently joined to a compound capable of providing a detectable signal; and determining the presence of cells to which said detectable signal compound is specifically bound.

Still another aspect of the invention is a peptide compound of at least 8 amino acids not more than about 30 amino acids, which peptide modulates CTL activity and consists of a sequence of at least 8 amino acids coming within the extended sequence defined above for use in a method of treatment of the human or animal body.

Preferred peptides for use in such a method of treatment of the human or animal body are:

(A)

    (a) peptides that modulate CTL activity and consist of at least 8 amino acids coming within the extended sequence


$aa^{55}$ G P E Y W D $aa^{62}$ $aa^{63}$ T $aa^{65}$ $aa^{66}$ $aa^{67}$ K $aa^{69}$ $aa^{70}$ $aa^{71}$

Q T  $aa^{74}$ R $aa^{76}$ $aa^{77}$ L $aa^{79}$ $aa^{80}$ $aa^{81}$ $aa^{82}$ $aa^{83}$ Y Y N Q S E A G

S H $aa^{94}$ $aa^{95}$ Q $aa^{97}$ M $aa^{99}$ G C D $aa^{103}$ G $aa^{105}$ D $aa^{107}$ R $aa^{109}$ L R

G $aa^{113}$ $aa^{114}$ Q $aa^{116}$ A Y D G


wherein:

    $aa^{55}$ is E or K;

    $aa^{62}$ is G, Q, E, or R;

    $aa^{63}$ is an acidic amino acid or amide thereof;

    $aa^{65}$ is Q, R or G;

aa$^{66}$ is I, N or K;

aa$^{67}$ is an aliphatic neutral amino acid;

aa$^{69}$ is an aliphatic neutral amino acid;

aa$^{70}$ is Q, H, S, N, or K;

aa$^{71}$ is an aliphatic neutral amino acid;

aa$^{74}$ is D, Y or H;

aa$^{76}$ is E or V;

aa$^{77}$ is D, S or N;

aa$^{79}$ is R or G;

aa$^{80}$ is T, I or N;

aa$^{81}$ is an aliphatic non-polar amino acid;

aa$^{82}$ is R or L;

aa$^{83}$ is G or R;

aa$^{94}$ is T or I;

aa$^{95}$ is a non-polar aliphatic amino acid of from 5 to 6 carbon atoms;

aa$^{97}$ is an aliphatic amino acid or W;

aa$^{99}$ is an aromatic amino acid;

aa$^{103}$ is a non-polar aliphatic amino acid of from 5 to 6 carbon atoms;

aa$^{105}$ is P or S;

aa$^{107}$ is G or W;

aa$^{109}$ is L or F;

aa$^{113}$ is Y or H;

aa$^{114}$ is H, Q, D, N, or R;

aa$^{116}$ is Y, D, S, F, or H;

with the proviso that the peptide is not a labelled peptide of the formula:

(i) R E T Q I C K A K

(ii) A Q T D R E aa$^{77}$ L R

where aa$^{77}$ is D, S or N;

(iii) G Y Y N Q S E A G S H T L Q aa$^{97}$

where aa$^{97}$ is S or R;

(iv) Y G C D V G P D G R;

(b) peptides as defined in (A)(a) above which consist of at least 8 amino acids coming within the sequence

G S H T [V, I, or L] Q R M Y G C D V G S D [W or G] R

F L R G Y H Q Y A Y D G.

or

Q E G P E Y W D (G or R) (E or N) T (R or Q) (K or N)

V K A (H or Q) S Q T  (H or D) R (V or E) (D, S or N) L

(G or R) (T or I) (L or A) (R or L) (G or R) Y Y N Q S E A,

wherein the amino acids in the parentheses indicate that any of one amino acids in the parentheses is present.

(B)

$$\text{G S H } aa^{94} \text{ } aa^{95} \text{ Q } aa^{97} \text{ M } aa^{99} \text{ G C D } aa^{103} \text{ G } aa^{105} \text{ D}$$

$$aa^{107} \text{ R } aa^{109} \text{ L R G } aa^{113} \text{ } aa^{114} \text{ Q } aa^{116} \text{ A Y D G;}$$

wherein
$aa^{94}$ is T or I;
$aa^{95}$ is a non-polar aliphatic amino acid of from 5 to 6 carbon atoms;
$aa^{97}$ is an aliphatic amino acid or W;
$aa^{99}$ is an aromatic amino acid;
$aa^{103}$ is a non-polar aliphatic amino acid of from 5 to 6 carbon atoms;
$aa^{105}$ is P or S;
$aa^{107}$ is G or W;
$aa^{109}$ is L or F;
$aa^{113}$ is Y or H;
$aa^{114}$ is H, Q, D, N or R;
$aa^{116}$ is Y, D, S, F or H; or

(C)

$$aa^{55} \text{ G P E Y W D } aa^{62} \text{ } aa^{63} \text{ T } aa^{65} \text{ } aa^{66} \text{ } aa^{67} \text{ K } aa^{69} \text{ } aa^{70}$$

$$aa^{71} \text{ Q T } aa^{74} \text{ R } aa^{76} \text{ } aa^{77} \text{ } aa^{79} \text{ } aa^{80} \text{ } aa^{81} \text{ } aa^{82} \text{ } aa^{83} \text{ Y Y N Q S E A}$$

wherein
$aa^{55}$ is E or K, particularly E;
$aa^{62}$ is G, Q, E or R, particularly R or G;
$aa^{63}$ is an acidic amino acid or amide thereof; including E and N, particularly E;
$aa^{65}$ is Q, R or G, particularly Q or R;
$aa^{66}$ is I, N, or K, particularly N or K;
$aa^{67}$ is an aliphatic neutral amino acid including V, M, S, C and Y, particularly V;
$aa^{69}$ is an aliphatic neutral amino acid including, A, T and P, particularly A;
$aa^{70}$ is Q, H, S, N or K, particularly Q or H:
$aa^{71}$ is an aliphatic neutral amino acid including S, A and T, particularly S;
$aa^{74}$ is D, Y or H, particularly D or H;
$aa^{76}$ is E or V;
$aa^{77}$ is D, S or N, particularly D;
$aa^{79}$ is R or G, particularly G;
$aa^{80}$ is T, I or N, particularly T or I;
$aa^{81}$ is an aliphatic non-polar amino acid including L or A, particularly L;
$aa^{82}$ is R or L, particularly R;
$aa^{83}$ is G or R, particularly G.

One preferred method of treatment in which such peptides can be used is a method of treatment that comprises inhibiting the cytolytic activity of a cytotoxic T-lymphocyte.

Another preferred method of treatment in which such peptides can be used is a method of treatment that comprises sensitizing MHC restricted cells to a cytotoxic T-lymphocyte. Particularly preferred peptides for use in a method of treatment are those of formula (C) above.

A further aspect of the invention is a peptide as defined in above conjugated to an agent which causes cytotoxicity, for use in a method of inhibiting cytolytic activity of a CTL (Cytotoxic T-lymphocyte) by irreversibly binding the CTL.

BRIEF DESCRIPTION OF THE DRAWINGS

Fig. 1 shows the minimum size peptide sequence required for inhibition of cytolysis by HLA-A2 specific CTL.

Fig. 2 shows the effect of pretreatment of CTL and of target cells on the inhibition of cytolysis by HLA-A2 specific CTL.

Fig. 3 shows the effect of peptide A2.98-113 on release of granules containing serine esterase during cytolysis of target cells by CTL.

Fig. 4 shows the consensus sequence of peptides which constitute the $\alpha_1$, $\alpha_2$, and $\alpha_3$ regions of a class I HLA molecule, as well as changes in these sequences in different specific HLA molecules.

Fig. 5 shows the effect of peptides from different HLA-A2 epitopes on cytolysis of target cells by CTL of different specificities.

Fig. 6 shows the sensitization of an HLA-Aw69 target cell to cytolysis by clone A2/B17 cells caused by peptide A2.56-69.

Fig. 7 shows the effect on sensitization of incubating target cells or clone A2/B17 cells with peptide A2.56-69.

DESCRIPTION OF THE SPECIFIC EMBODIMENTS

Methods and compositions are provided for modulating the effect of cytotoxic T lymphocytes or diagnosing the presence of CTL's of a predetermined specificity. The compositions are peptide sequences cross reactive with a polymorphic portion of the $\alpha_1$ or $\alpha_2$ domains of Class I major histocompatibility antigens, particularly the C-terminal portion of the $\alpha_1$ domain and the N-terminal portion of the $\alpha_2$ domain. These major histcompatibility antigens are classified as Groups A, B, and C, having a substantial number of subclassifications within these groups, where the different Class I antigens are able to bind specifically to complementary CTL's, which are restricted by such antigens. Of particular interest for the $\alpha_1$ domain is the amino acid sequence from positions 55 to 85, more particularly 55 to 80, usually 55 to 70, desirably including within the sequence a tetrapeptide DGET, GETR, DRET, or YWDG. Of particular interest for the $\alpha_2$-domain is the amino acid sequence from positions 90 to 120, more particularly 94 to 116, desirably including within the sequence a tetrapeptide SDWR or SDGR.

The peptides of interest which will serve as the receptor binding peptide will have at least 8 amino acids, usually at least 10 amino acids, and usually not more than about 30 amino acids, more usually not more than about 24 amino acids, desirably having about 12 to 21 amino acids. The amino acid sequence will usually not differ from a naturally occurring sequence by more than 2 amino acids, or mutations, e.g., deletions or insertions, more usually by not more than about 1 amino acid. The sequence employed will usually be from the polymorphic regions of the C-terminal half of the $\alpha_1$ domain or the N-terminal half of the $\alpha_2$ domain of the MHC antigen of the host of the MHC restricted T-cells, particularly an HLA-A group antigen.

For the most part, the amino acid sequences for the $\alpha_1$ region will come within the following formula:

$$\text{aa}^{55}\ G\ P\ E\ Y\ W\ D\ \text{aa}^{62}\ \text{aa}^{63}\ T\ \text{aa}^{65}\ \text{aa}^{66}\ \text{aa}^{67}\ K\ \text{aa}^{69}$$
$$\text{aa}^{70}\ \text{aa}^{71}\ Q\ T\ \text{aa}^{74}\ R\ \text{aa}^{76}\ \text{aa}^{77}\ L\ \text{aa}^{79}\ \text{aa}^{80}\ \text{aa}^{81}\ \text{aa}^{82}$$
$$\text{aa}^{83}\ Y\ Y\ N\ Q\ S\ E\ A$$

$\text{aa}^{55}$ is E or K, particularly E:

$\text{aa}^{62}$ is G, Q, E or R, particularly R or G;

$\text{aa}^{63}$ is an acidic amino acid or amide thereof; including E and N, particularly E;

$\text{aa}^{65}$ is Q, R or G, particularly Q or R;

$\text{aa}^{66}$ is I, N or K, particularly N or K;

$\text{aa}^{67}$ is an aliphatic neutral amino acid including, V, M, S, C and Y, particularly V,

$\text{aa}^{69}$ is an aliphatic netural amino acid including, A, T and P, particularly A;

$\text{aa}^{70}$ is Q, H, S, N or K, particularly Q or H:

$\text{aa}^{71}$ is an aliphatic neutral amino acid including S, A and T, particularly S;

$\text{aa}^{74}$ is D, Y or H, particularly D or H;

$\text{aa}^{76}$ is E or V;

8

$aa^{77}$ is D, S or N, particularly D;

$aa^{79}$ is R or G, particularly G;

$aa^{80}$ is T, I or N, particularly T or I;

$aa^{81}$ is an aliphatic non-polar amino acid including L or A, particularly L;

$aa^{82}$ is R or L, particularly R;

$aa^{83}$ is G or R, particularly G.

The peptide may be extended by bonding at one or both termini with other than a wild-type sequence for the HLA-A2 antigen.

Of particular interest is a sequence or sequence fragment of at least 8 amino acids of the sequence:

Q E G P E Y W D (G or R) (E or N) T (R or Q) (K or N) V

K A (H or Q) S Q T (H or D) R (V or E) (D, S or N) L (G

or R) (T or I) (L or A) (R or L) (G or R) Y Y N Q S E A

For the most part, the amino acid sequences for the $\alpha_2$ domain will come within the following formula:

91                                                       100

G S H $aa^{94}$ $aa^{95}$ Q $aa^{97}$ M $aa^{99}$ G C D $aa^{103}$ G $aa^{105}$ D

$aa^{107}$ R $aa^{109}$ L R G $aa^{113}$ $aa^{114}$ Q $aa^{116}$ A Y D G

$aa^{94}$ is T or I, particularly T;

$aa^{95}$ is a non-polar aliphatic amino acid of from 5 to 6 carbon atoms, particularly I, L or V;

$aa^{97}$ is an aliphatic amino acid, either polar or nonpolar, or W, including R, M, N, S, I, and W, particularly R;

$aa^{99}$ is an aromatic amino acid, Y or F, particularly Y;

$aa^{103}$ is a non-polar aliphatic amino acid of from 5 to 6 carbon atoms, particularly I, V, or L, more particularly V;

$aa^{105}$ is P or S, particularly S;

$aa^{107}$ is G or W, particularly W;

$aa^{109}$ is L or F, particularly F;

$aa^{113}$ is Y or H, particularly Y;

$aa^{114}$ is H, Q, D, N or R, particularly H;

$aa^{116}$ is Y, D, S, F or H, particularly Y or D.

The peptide may be extended by bonding at one or both termini with other than a wild-type sequence for the A2 antigen.

Of particular interest is a sequence or sequence fragment that consists of at least 8 amino acids of the sequence:

G S H T [V, I, or L] Q R M Y G C D V G S D [W or G] R F

L R G Y H Q Y A Y D G.

Where there are two or more amino acids indicated at the same site, any of the indicated amino acids may be present.

The region of particular interest will be the region from amino acid positions 100 to 116.

The classes of amino acids are designated as follows:

aliphatic

non-polar G, A, P, L, I, V

polar

neutral C, S, T, M, N, Q

acidic D, E

basic K, R

9

aromatic F, H, W, Y

The peptides may be prepared in a variety of ways. Conveniently, they can be synthesized by conventional techniques employing automatic synthesizers, such as the Beckman, Applied Biosystem Inc., or other useful peptide synthesizer apparatus, or may be synthesized manually. Alternatively, DNA sequences can be prepared which encode the particular peptide and may be cloned and expressed to provide the desired peptide. In this instance a methionine may be the first amino acid.

The peptides may also be isolated from natural sources and purified by known techniques, including, for example, chromatography on ion exchange materials, separation by size, immunoaffinity chromatography and electrophoresis. As used herein, the term "a substantially pure preparation of peptide compound" means a preparation of the peptide which is usually greater than about 70% free of materials with which the polypeptide is naturally associated, and preferably greater than about 80% free of these materials; these materials, however, excludes materials with which the peptide may be mixed in the preparation of pharmaceutical compositions.

The sequences may be modified in a variety of ways depending upon their ultimate purpose. Different N- or C- terminal groups may be introduced which allow for linking of the peptide to solid substrates or other molecules. In a synthetic procedure, any molecule may be introduced at the N- or C-terminus which would allow for subsequent reaction, depending upon the purpose for which the peptide is prepared.

For diagnostic purposes, a wide variety of labels may be linked to the terminus, which may provide, directly or indirectly, a detectable signal. For example, fluorescers may be introduced at the terminus or other molecules which provide a linkage to labels such as fluorescers, enzymes, particles, or the like. For example, linkage may be introduced at the terminus, e.g., biotin, which will bind to an avidin conjugate with enzymes or fluorescers. Alternatively, various reactive sites may be introduced at the terminus for linking to particles, solid substrates, macromolecules, or the like. For example, an internal amino moiety of a growing chain bound to a solid substrate with the intermediate side groups protected, may be conjugated with methyldithiobenzoic acid (MDTB). The free mercaptan group may then be used for conjugating with activated olefins. Thus, proteins, such as serum albumin, keyhole limpet hemocyanin, bovine $\beta$-globulin, or the like, may be conjugated to the peptide to provide for an immunogen to produce antibodies to the peptide for use in immunoassays, for affinity chromatography, or the like. Alternatively, the peptide can be bonded to another polypeptide by preparing a DNA sequence which has the peptide at the N-terminus, C-terminus or internal to the protein, so as to provide a fused protein which includes the binding peptide of interest. In this manner, fused proteins may be produced which have enzymatic activity, which enzymatic activity may be modulated by macromolecules, e.g., antibodies, binding to the peptide of interest. Thus, the peptides of the subject invention may be modified in a wide variety of ways for a variety of end purposes while still retaining biological activity.

The subject peptides may also be used in combination with antigenic peptides or proteins of interest to activate CTL's. Thus, the subject peptides may be bound to a protein, either directly or indirectly, so as to be able to present two epitopes to the CTL to which the CTL may bind and be activated. Of particular interest, is where the subject peptides may be bound to a liposome or a bilayer lipid membrane in conjunction with a peptide or protein providing the other determinant site.

Various techniques are available for joining a peptide or protein to a lipid, particularly a phospholipid to provide for the presence of the peptide or protein on the liposome surface. Phosphatidyl choline, phosphatidyl ethanolamine, or other lipid may be used with a bifunctional linking agent, such as MBSE, glutaraldehyde, methyldithiobenzoic acid, or the like. The formation of liposomes with conjugated proteins finds ample support in the literature, see, for example, U.S. Patent Nos. 3,887,698; 4,261,975: and 4,193,983. The modified peptide or protein is combined with the lipids in an aqueous medium and sonicated to provide the desired liposomes. The liposomes may then be harvested and used in the ways indicated.

The subject peptides, by themselves, or in combination with other peptides or proteins, may be used for diagnosing the presence of CTL's which bind to a subject peptide or the combination of a subject peptide and other peptide or protein. In this manner, conjugates of the subject peptide and the antigenic peptide or protein can be prepared by employing linking agents as described previously. Alternatively, the subject peptide and the antigenic peptide may be bound to a solid surface, such as a particle, container surface, or the like. If desired, the subject peptide and antigenic peptide or protein may be conjugated to a particle or protein which is fluorescent. The binding of the particle or protein will allow for sorting and counting in a fluorescence activated cell sorter.

The subject peptides may also be used for modulating CTL activity in the mammalian host. The modulation may be by inhibiting CTL activity or by sensitizing target cells. This can be achieved by employing apheresis, where the patient's blood is withdrawn from the patient and circulated through a device in which the peptide is present, either bound to the surface, to remove CTL's active with the subject peptide or in a physiologically acceptable medium to bind to the CTL's and inhibit their activity. Alternatively, the subject peptides may be administered to a host intravascularly, in either an artery or vein, to provide for inhibition or stimulation of the CTL.

Examples of inhibitory peptides are presented infra (see Examples 2 and 9), which are derived from both the $\alpha_1$ and $\alpha_2$ domain of HLA-A2. In each case the sequence of the inhibitory peptide correlates with the epitope specificity of the CTL. Moreover, as shown in Example 4, inhibition is mediated by an octapeptide, and occurs by peptide binding to the CTL and not the target cell (see Example 5). Since the inhibitory capacity of the individual peptides correlates with CTL specificity, it seems likely that these peptides inhibit by binding to the variable T cell receptor.

An example of a peptide which stimulates cytolysis of a HLA-class I bearing target cells by alloreactive CTL is presented in Example 10, infra. The simplest interpretation of the results in Examples 10-12 is that the HLA-A2/B17 specific CTL recognize the A2 56-69 peptide in the context of HLA-Aw69 as a restriction element. This implies that the peptide is binding to the HLA-Aw69 molecule. The data and interpretation are similar to those obtained in the influenza (Bastin et al., J. Exp. Med., 165:1508 (1987); Gotch et al., Nature 326:881 (1987)) and xenogeneic systems (Maryanski et al., Nature 324:578 (1986)), and demonstrate that alloreactive CTL can recognize Class I derived peptides in a Class I restricted fashion. However, the quantities of peptide required to cause sensitization are significantly larger than those reported in other studies. Although the molecular basis for this is as yet unknown, one possible explanation involves the relative handling of exogenous versus endogenous molecules. For example, HLA is an endogenous molecule, and the exogenous HLA peptides may have to compete with endogenous HLA peptides. Another alternative is that the A2.56-69 peptide may not include all of the residues required for high affinity binding to the target cell.

Two of the CTL epitopes from which the peptides described in the Examples, infra. are derived, are situated in very different parts of the HLA-A2 molecule. Residues 62-65 are in an alpha helix which forms part of the peptide binding site (Bjorkman et al., Nature 329:506 (1987)), which is itself thought capable of binding alpha helical peptides. As shown in the Examples, peptides in this region can either inhibit or induce cytolysis. In the induction of cytolysis, it is possible that the peptide may bind to a target cell HLA class I antigen and thereby create a structure which is recognized by the CTL. For example, in the case of A2.56-69 peptide conferring sensitivity to clone A2/B17 cells on HLA-Aw69 cells, the bound peptide presumably substitutes for the $\alpha$ helix of the $\alpha_1$ domain, since HLA-Aw69 and HLA-A2 have identical $\alpha_2$ domains.

In contrast, residue 107 is part of a turn between two strands of $\beta$ structure at some distance from the alpha helices and peptide binding region (Bjorkman et al., supra.) The A2.98-113 peptide may maintain elements of this structure in solution and have little affinity for the peptide binding site of Class I molecules. This interpretation would explain the observation in the Examples that peptides corresponding to this region are inhibitors of HLA-A2 directed cytolysis, but cannot induce cytolysis.

As already indicated, the peptide may be present by itself, or in combination with an antigen thereby providing a different determinant site of interest. Depending upon whether only the subject peptide is included, or the peptide in combination with other peptides, activation or inhibition can be achieved.

If irreversible inhibition is desired, the conjugate of the subject peptide with the antigen may be joined to a cytotoxic agent, joined to liposomes containing cytotoxic agents, or joined to a specific monoclonal antibody or immunoglobulin, whereby binding of the conjugate to the CTL will result in the complement mediated lysis of the CTL.

The subject peptides, by themselves or as conjugates, may be prepared as formulations in pharmaceutically acceptable media, for example saline, PBS, and glucose, generally at a pharmacologically effective dose, the concentrations of which will be determined empirically in accordance with conventional procedures for the particular purpose. The additives may include bactericidal agents, stabilizers, buffers, or the like. The amount administered to the host will vary depending upon what is being administered, the purpose of the administration, such as prophylaxis or therapy, whether inhibition or activation is desired, the state of the host, the manner of administration, and the like. In order to enhance the half-life of the subject peptide or subject peptide conjugates, the peptides may be encapsulated, introduced into the lumen of liposomes, prepared as a colloid, or other conventional technique may be employed which provides an extended lifetime of the peptides.

The following examples are offered by way of illustration and not by limitation.

EXAMPLES

Example 1

Preparation of Peptides Derived From HLA-A2

Four peptides were prepared by conventional synthetic methods using standard solid-phase methods. See Erickson & Merrifield in: The Proteins Vol. 2, 3rd edition (eds. Neurath, H. & Hill, R.L.) p. 255-527 (Academic Press, N.Y. 1970), which is hereby incorporated herein by reference. Three of the peptides had amino acids from the $\alpha_2$ domain and one of the peptides had amino acids from the $\alpha_2$ domain of a HLA-A2 antigen. The four peptides had the following compositions and designations:

A2.56-69 G P E Y W D G E T R K V K A
A2.94-112 T L Q R M Y G C D V G S D W R F L R G
A2.98-113 M Y G C D V G S D W R F L R G Y
Aw.68 98-113 M Y G C D V G S D G R F L R G Y

The designations indicate the major histocompatability antigen from which the peptide is derived, and the position of the amino acids in the antigen.

Example 2

Inhibition of HLA-A2 Specific CTL by Peptides Derived from HLA-A2.98-113 and HLA-A2.94-112

Peptides prepared as in Example 1, i.e., those corresponding to HLA-A2.56-69, HLA-A2.94-112, HLA-A2.98-113, and HLA-Aw 68.98-113, were preincubated for 30 min. with 1-3 x $10^3$ CTLs before addition of $10^3$ CPM of $^{51}$Cr-labeled B-lymphoblastoid target cells. The cytotoxicity assay was then performed as described by Clayberger et al., J. Exp. Med. (1984) 162:1709-1714; and Reiss et al., Proc. Natl. Acad. Sci. USA (1980) 77:5432-5436, which are hereby incorporated herein by reference.

In the first study, the CTL cell line was AJY, a long term CD8$^+$ CTL line specific for HLA-A2, and the target cell was the B-lymphoblastoid cell line JY (HLA-A2, B7). In the second study the CTL was PWSB, a bulk culture with reactivity against HLA-B17 and the target was FMB, which expresses HLA-A1, A32, B17. In each case the percentage of specific release obtained in the absence of peptide was determined. The lower amount of specific release in the second study potentially made cytolysis more sensitive to inhibition. Stocks of peptides at 1 mg/ml in PBS were diluted to give final concentrations in the assay as indicated in Table 1. As a control inhibitor, the monoclonal antibody PA2.6 which is directed against the monomorphic determinant of HLA-A, B, C molecules was used (Reiss et al., supra: McMichael, J. Exp. Med. (1980) 152:195s-203s). The peptides employed were A2.98-113, A2.94-112, Aw68. 94 112 and A2.56-69. The following table indicates the results.

Table 1

| | Concentration $\mu$g/ml | % Specific Lysis | | | |
| --- | --- | --- | --- | --- | --- |
| | | A2.98-113 | A2.94-112 | Aw68.94-112 | A2.56-69 |
| Trial 1. | 160 | 0 | 3 | 52 | 51 |
| CTL = AJY | 80 | 4 | 20 | 45 | 38 |
| Target = JY | 40 | 18 | 35 | 63 | 61 |
| Trial 2. | 160 | 27 | 35 | 28 | 20 |
| CTL = PWSB | 80 | 29 | 32 | 30 | 27 |
| Target = FMB | 40 | 30 | 34 | 35 | 31 |

In the first case, the percentage specific release obtained in the absence of peptide was about 54, while in the second case it was about 28.

The above results with CTL which are restricted by the HLA-A2 antigen, show inhibition of specific cytotoxicity. With CTL's not restricted by A2, lysis of random target cells occurs with the results approximating the standard specific release obtained in the absence of peptide. These results suggest that the tryptophan at position 107 may be critical. Peptide A2.98-113 and peptide Aw68.98-113 are homologous except for the substitution of glycine for tryptophan at this position; this substitution resulted in a loss of inhibition of cytolysis by HLA-A2 specific CTL.

The results of treatment of peptide A2.98-113 with different proteases, i.e., trypsin or chymotrypsin, allow the suggestion that arginine 108 is of importance, but that peptides 109-113 are not critical. The major sites of action of trypsin and chymotrypsin are Arg, Lys, and Trp, Phe, Tyr, respectively. Chymotryptic, but not tryptic, cleavage of the peptide reduced the inhibitory activity (results not shown).

Example 3

Effect of Specificity of CTL and Target Cell on Inhibition of Cytolysis Caused by HLA-Derived Peptides

A number of different CTL cell lines were studied, where the specificity of the cell lines were varied. The results shown in Table 2 indicate that only where the CTL's and the target cells share A2 specificity do the A2-derived peptides provide inhibition.

Table 2

Specificity of CTL Tested for Inhibition by Peptides

| CTL | Specificity of CTL | Target Cell | Target Molecule | Inhibition of Lysis by Peptide | | | |
|---|---|---|---|---|---|---|---|
| | | | | A2.98-113 | A2.94-112 | Aw68.98-113 | A2.56-69 |
| Line AJY | A2 | JY | A2 | + | + | - | - |
| Line PJY | A2 | JY | A2 | + | + | - | - |
| Clone A20.1 | A2 | JY | A2 | + | + | - | - |
| Clone AI.10 | A2 | JY | A2 | + | + | - | - |
| Clone AI9.1 | A2,Aw68, Aw69 | JY | A2 | + | + | - | - |
| Line PWSB | A2,B17 | JY | A2 | + | + | - | - |
| Line PWSB | A2,B17 | FMB | B17 | - | - | - | - |
| Clone AL8.1 | Aw68,Aw69 | LB | Aw68 | - | - | - | ND |
| Clone A15.1 | Aw69 | IDF | Aw69 | - | - | - | ND |
| Line CJY | Dr6 | JY | DR6 | - | ND | - | - |
| Line CJY | Dr6 | DAUDI | DR6 | - | ND | - | - |

The specificity of the CTL is based upon analysis of the pattern of killing on a panel of B lymphoblastoid cell lines and by patterns of inhibition with monoclonal antibodies. ND indicates not done. CTL used were from four different donors.

Example 4

Minimum Peptide Sequence Required for Inhibition of HLA-A2 Specific CTL

The minimum peptide sequence required for inhibition of cytolysis by HLA-A2 specific CTL was determined by examining the effect of size on the inhibition.

A series of peptides which started at positions 98-104 and ended at position 108 of HLA-A2 or HLA-Aw68 were synthesized. The effect of these peptides on cytolysis of JY cells (HLA-A2, B7, DR4,6) by

14

seventeen different HLA-A specific lines or clones were tested. The HLA-A2 specific lines or clones were generated as described in Clayberger et al., supra. Peptides (200 mg/ml) were preincubated with 1-3 x $10^3$ CTL for 30 minutes prior to addition of $10^3$ CPM of $^{51}$Cr-labeled target cells. The peptides were present throughout the cytotoxicity assay which was performed as described in Clayberger et al., supra, and in Krensky et al., Proc. Natl. Acad. Sci. USA 79:2365 (1982), which are hereby incorporated herein by reference. Peptides were prepared as stock solutions at 1 mg/ml in phosphate buffered saline and diluted in complete medium (MEM supplemented with 10% calf serum) to give the final concentration used.

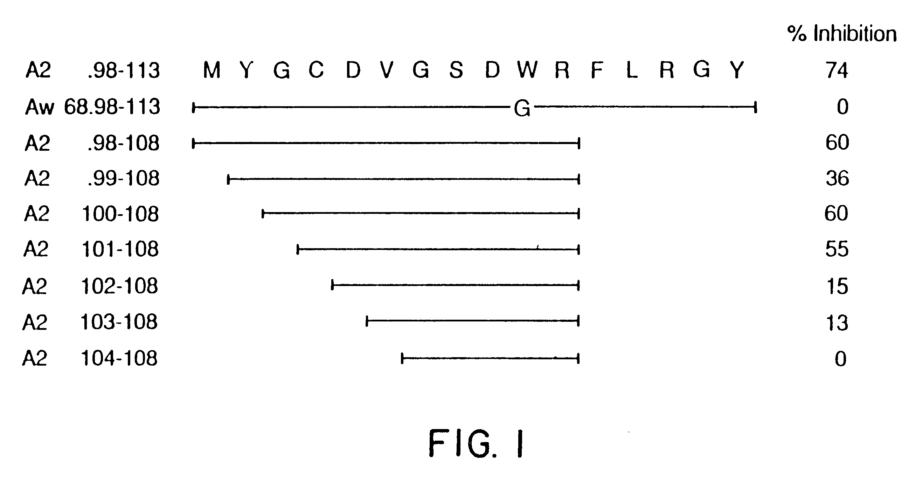

The results on the inhibition of cytolysis by CTL-A2 is shown in Fig. 1A, where inhibition is expressed as (1-[specific cytolysis in the presence of peptide/specific cytolysis in the absence of peptide]) x 100.

As seen in the figure, peptide 104-108 did not inhibit, peptides 102-108 and 103-108 caused weak inhibition, and the remaining peptides caused good inhibition of cytolysis. Thus, an octapeptide comprising residues 101-108 was sufficient to cause the inhibitory effect.

A major decrease in the inhibitory effect occurs with loss of the cysteine at position 101. This loss may be due to the loss of disulfide cross-linking of two peptide molecules when cysteine 101 is absent.

## Example 5

### Locus of Action of Peptide A2.98-113

The locus at which peptide A2.98-113 interacts to cause an inhibitory effect on HLA-A2 specific CTL mediated cytolysis, i.e., with the CTL and/or with the target cell, was determined as follows.

The CTL (1 x $10^6$ CTL-A2) and/or the target cells ($^{51}$Cr-labeled JY target cells) were incubated with 100 μg of A2.98-113 for 30 min. at 37°C, or alternatively with the control peptide, Aw68.98-113. The sequences of these peptides are presented in Example 1. As an additional control, the cells were incubated with complete medium minus peptide. Following the incubation, the cells were washed three times in complete medium, and tested in a $^{51}$Cr-release assay (see Example 2).

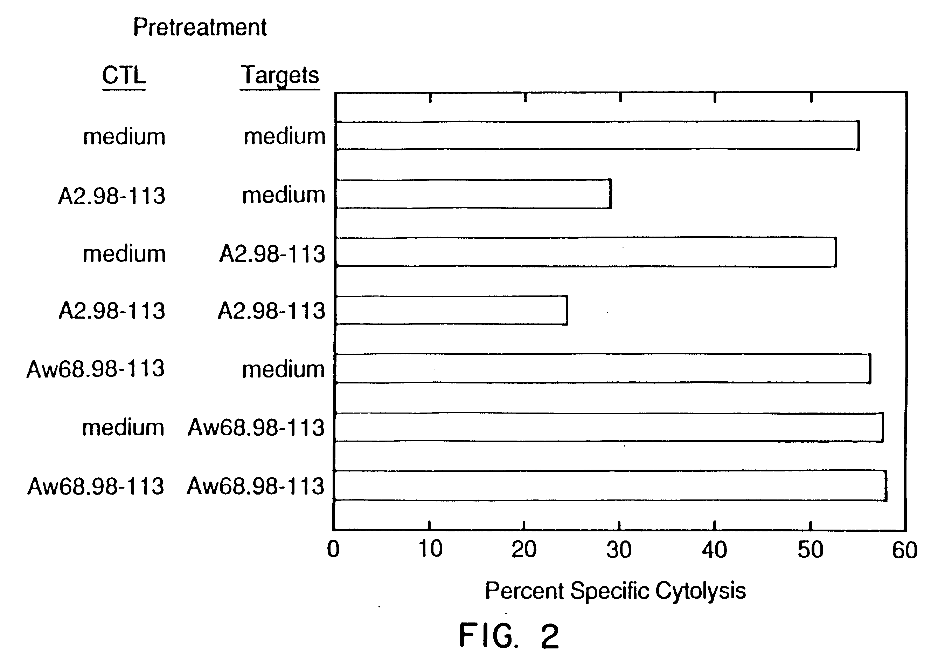

The results are presented in Fig. 2, where it may be seen that lysis was inhibited when the CTL, but not the target cells, were pretreated with A2.98-113. Inhibitory effects were not observed when CTL or target cells were pretreated with the control peptide, Aw68.98-113.

## Example 6

### Mechanism of Inhibition of CTL by A2.98-113

### Effect on CTL Viability

To determine whether CTL were inhibited due to their autolysis induced by A2.98-113, either $^{51}$Cr-labeled CTL-A2 cells or unlabeled CTL-A2 cells were incubated with the peptide for 6 hours at 37°C in complete medium. During the 6 hour incubation there was no detectable decrease in cell viability as judged by exclusion of trypan blue or by $^{51}$Cr-release (results not shown).

## Example 7

### Mechanism of Inhibition of CTL by A2.98-113

### Effect on Release of Granules Containing Serine Esterase

The effect of A2.98-113 on release of granules containing serine esterase during cytolysis of target cells by CTL was determined as follows.

The specificity of release was determined by incubating 3 x $10^5$ HLA-A2 specific CTL with JY cells (HLA-A2; B7; Dr4,6) or IBW4 cells (HLA-A3; B35; DR1) for 2 hours in V bottom microtiter wells. The ratios of CTL:target cells were 1:0.01, 1:0.05, 1:0.10, 1:0.5, and 1:1. After the incubation, the plates were spun at 1000 RPM for 2 minutes, and the supernatant was assayed for serine esterase activity essentially as described in Young et al., Cell 47:183 (1986), which is hereby incorporated herein by reference. The reaction mixtures consisted of 20 μl of supernatant plus 200 μl of substrate (2 x $10^{-4}$ M N-benzyloxycarbonyl-L-lysine thiobenzyl ester, 2.2 x $10^{-4}$ M nitrobenzoic acid, 0.1M Tris-HCl, pH 8.0). After 30 min. at 37°C, the absorbance was determined at 410 nm. Total serine esterase activity was determined by substituting 0.01% Triton X-100 for stimulator cells.

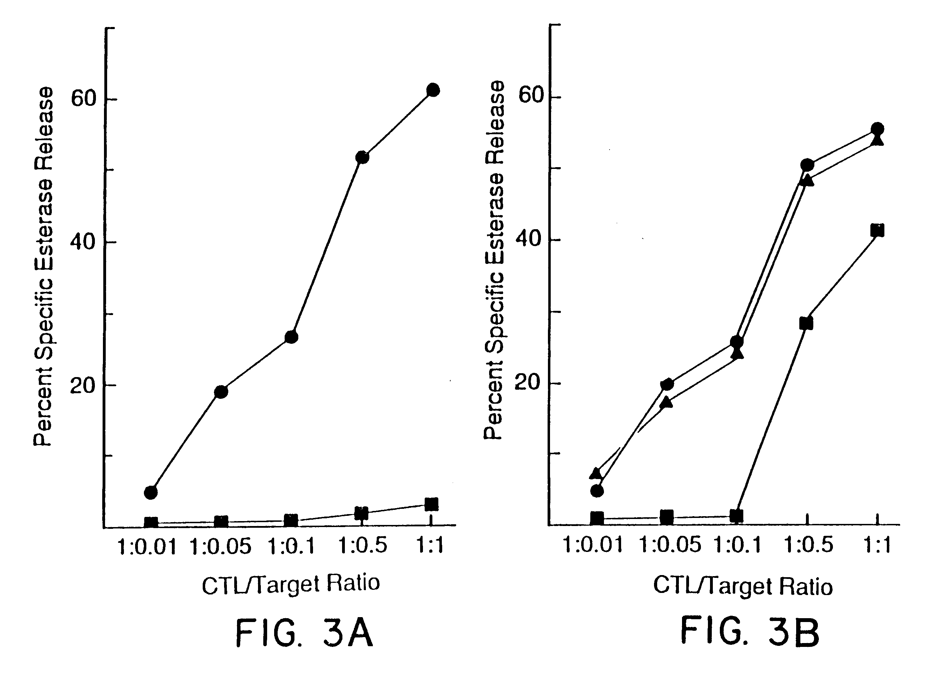

The results, shown in Fig. 3A, indicate that release of the granules occurred when the HLA-A2 specific CTL were incubated with JY cells (closed circles), but not when the HLA-A2 specific CTL were incubated with IBW4 cells (closed squares).

The effect of peptide A2.98-113 on release of granules containing serine esterase was determined in a similar fashion, except that the HLA-A2 specific CTL were preincubated with 100 $\mu$g of peptide, either A2.98-113 or Aw68.98-113, or with only complete medium, for 30 min. at 37°C prior to the addition of JY target cells at ratios of CTL:target cells of 1:0.01, 1:0.05, 1:0.1, 1:0.5 and 1:1.

As seen in Fig. 3B, complete inhibition of esterase release was seen with 100 $\mu$g/ml of A2.98-113 at an effector-to-target ratio of 1:0.1 (closed squares). The control peptide Aw68.98-113 had no effect on esterase release (closed triangles), since release in this case was equal to that obtained with control cells preincubated with complete medium (closed circles).

These results, in conjunction with those in Example 5 indicate that the A2.98-113 peptide blocks events which occur early in T cell activation by binding directly to the CTL. This binding may be to the antigen receptor.

Example 8

Isolation of CTL Specific for the Epitope Shared by HLA-A2 and HLA-B17, for HLA-B17, and for HLA-2

CTL with the various specificities were derived from the peripheral blood lymphocytes of a normal donor (HLA-A3; B7; DR6) essentially as described by Clayberger et al. (1985), supra. For CTL specific for the epitope shared between HLA-A2 and HLA-B17, the cells were stimulated in primary culture with the irradiated (10,000R) B-lymphoblastoid cell line Mag (HLA-A26,33; B17,51) and cloned using the SB cell line (HLA-A1,2; B17,44; DR2,6) as stimulators. CTL specific for B17 were derived from the same primary culture, but were cloned using the SH cell line (HLA-A3,w33; B7,17(w57)) as stimulators. HLA-A2 specific CTL were derived from cell stimulated in primary culture with the JY cell line and cloned using the Herluff cell line (HLA-A2; B12,35; DR4,7) as stimulators. The fine specificity of these CTL clones was assessed using a panel of 11 targets expressing HLA-B17, 8 targets expressing HLA-A2 and 15 targets with unrelated HLA molecules. Multiple clones of the desired specificities were obtained. An individual clone which caused cytolysis of both HLA-A2 type target cells and HLA-B17 type target cells was designated clone A2/B17. The cytolysis of target cells of clone A2/B17 was inhibited by antibody MA2.1. A second clone, which lysed all HLA-B17 target cells but no others was designated B17. A third clone, which lysed all HLA-A2 target cells but no others was designated CTL-A2.

The target specificity of clone A2/B17 and the finding that cytolysis by this clone was blocked by monoclonal antibody MA2.1 indicates that cells of clone A2/B17 recognize the epitope shared by HLA-A2 and HLA-B17.

Example 9

The Effect of Peptides from Different HLA-A2 Epitopes on Cytolysis of Target Cells by CTL of Different Specificities

Examples 2-7, supra, have involved the effects of peptides derived from the region around tryptophan 107 in the $\alpha_2$ domain. This residue, which is on a bend between two strands of $\beta$ pleated sheet (Bjorkman et al., (1987), supra), is critical for a major serologic epitope of HLA-A2. Salter et al., J. Exp. Med. 166:283 (1987); Layet et al., J. Immunol. 138:2197 (1987).

Another important epitope involves residues 62-65 of the $\alpha$ helical region of the $\alpha_1$ domain. Bjorkman et al., supra. This epitope was originally defined by the monoclonal antibody MA2.1 (McMichael et al., Hum. Immunol. 1:121 (1980)), and is shared by all known subtypes of HLA-A2 and HLA-B17 (Ways and Parham, Biochem. J. 216:423 (1983)). A comparison of the amino acid sequence of HLA-A2 and HLA-B17 and eight other HLA-A,B,C proteins showed that only the glycine residue at position 62 is unique, suggesting that this residue contributes to a shared determinants (Ways et al., J. Immunol. 137:217 (1986)).

Peptides derived from the above two regions were examined for their inhibitory effect on cytolysis of target cells by CTL with different HLA specificities, i.e., those of clone A2/B17, clone CTL-A2, and clone B17 (see Example 8, supra). CTL were incubated with the following peptides: A2.56-69, Aw68.56-69, A2.98-113, or Aw68.98-113.

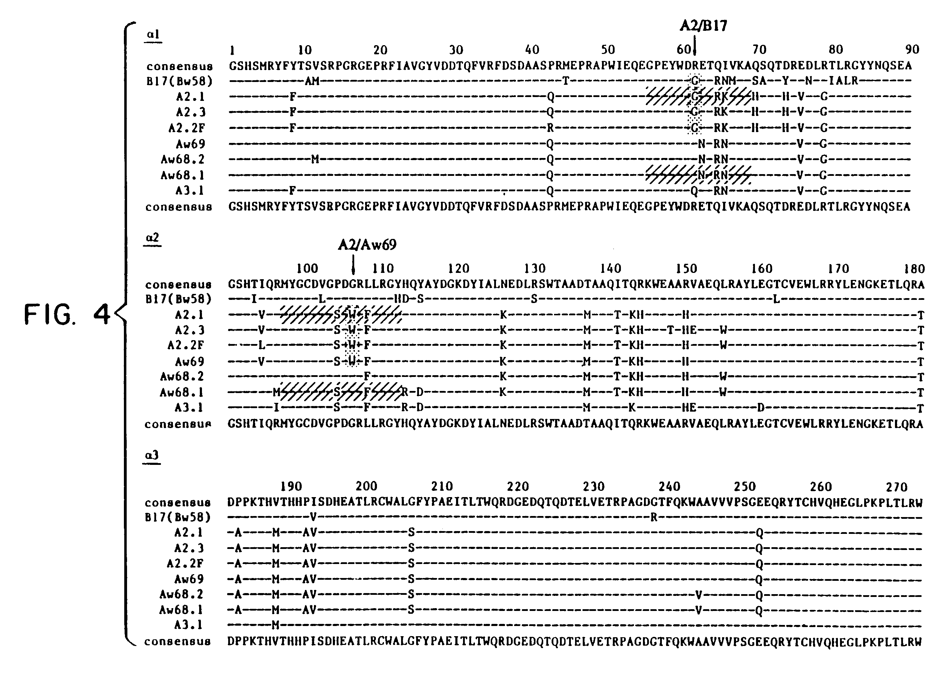

The epitopes studied and peptides used in the study are shown in Fig. 4, where the protein sequences in the three extracellular domains ($\alpha_1$, $\alpha_2$ and $\alpha_3$) of eight HLA-A,B molecules are shown using the standard one letter amino acid code. The sequence of HLA.Bw58 subtype of HLA-B17 is from Ways et al., J. Biol. Chem. 260:11924 (1985), that of HLA-A3.1 is from Strachen et al., EMBO J 3:887 (1984), and the remaining sequences of the HLA-A2/28 family are from Holmes et al., J. Immunol. 139:936 (1987). Peptides A2.56-69 and Aw68.56-69, and A2.98-113 and Aw68.98-113, which are derived from $\alpha_1$ and $\alpha_2$, respectively, are indicated by cross-hatching. The two residues found to be critical for the epitopes shared by subtypes of HLA-A2 and HLA-B17 (glycine 62) and subtypes HLA-A2 and HLA-Aw69 (tryptophan 107) are indicated by stippling and the vertical arrows. The consensus sequence is derived from a total of 23 HLA-A,B,C sequences.

The CTL were incubated with peptides at concentrations of 100 $\mu$g/ml, 200 $\mu$g/ml, or 300 $\mu$g/ml. Control samples were incubated in the absence of peptide. The final molar concentrations of peptides used in the assay at 100 $\mu$g/ml were 4.9 x $10^{-5}$ M for A2.98-113; 5.2 x $10^{-5}$ M for Aw68.98-113; 5.9 x $10^{-5}$ M for A2.56-69; and 5.9 x $10^{-5}$ M for Aw68.56-69. The CTL cells were incubated with the peptides for 20 min. prior to the addition of $10^3$ $^{51}$Cr-labeled T7529 cells (HLA-Aw33; B17(w58); DR6) or JY cells (HLA-A2; B17; DR4,6). In all cases, the effector-to-target ratios were 1:1.

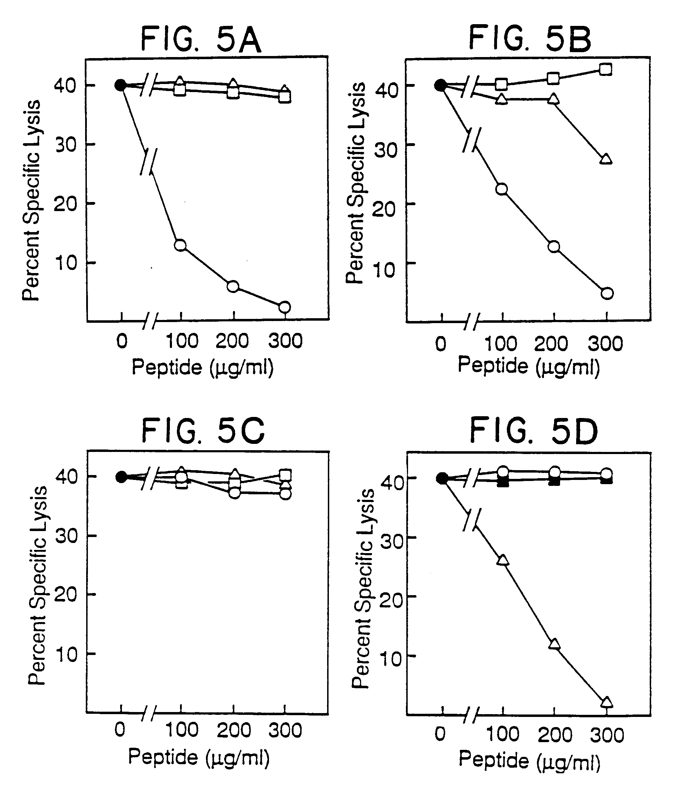

The results on cytotoxicity, as measured by $^{51}$chromium release from the target cells, is shown in Fig. 5. Figures 5A and 5B show the results of the effects of the peptides on cells of clone A2/B17; Fig. 5C shows the effects on cells of clone B17, and Fig. 5D on CTL-A2. The peptides are indicated as follows: (open circles) A2.56-69; (open squares) Aw68.56-69; (open triangles) Aw.98-113; and (closed squares) Aw68.98-113.

Peptide A2.56-69, which encompasses the shared serologic epitope, specifically inhibited the killing of both HLA-A2 and HLA-B17 expressing target cells by clone A2/B17 cells. In contrast, this peptide had no effect upon the lysis of HLA-B17 expressing cells by clone B17 cells. Clone A2/B17 cells were not inhibited by a peptide derived from residues 56-69 of HLA-Aw68.1, or by a series of unrelated peptides. The A2.98-113 peptide did not affect the lysis of HLA-B17 expressing targets by clone A2/B17 cells, but some inhibition was observed at high concentrations with HLA-A2 expressing targets. This difference indicates that the epitopes of HLA-A2 and HLA-B17 recognized by clone A2/B17 cells are not precisely the same.

These results show that the capacity of peptides to inhibit alloreactive CTL is not restricted to the region involving residues 101-108 of the $\alpha_2$ domain, and that they may be derived from a second epitope of HLA-A2.

The discrepancy of the results achieved with peptide A2.56-69 using clone A2/B17, and those with the PWSB cell line (see Table 2) with respect to the inhibitory effect of this peptide may be explained by the polyclonal nature of the PWSB cells. That is, the PWSB line probably is a mixture of CTL's including individual clones specific for HLA-A2 or HLA-B17.

Example 10

Sensitization of Target Cells to CTL caused by a HLA-2 Derived Polypeptide

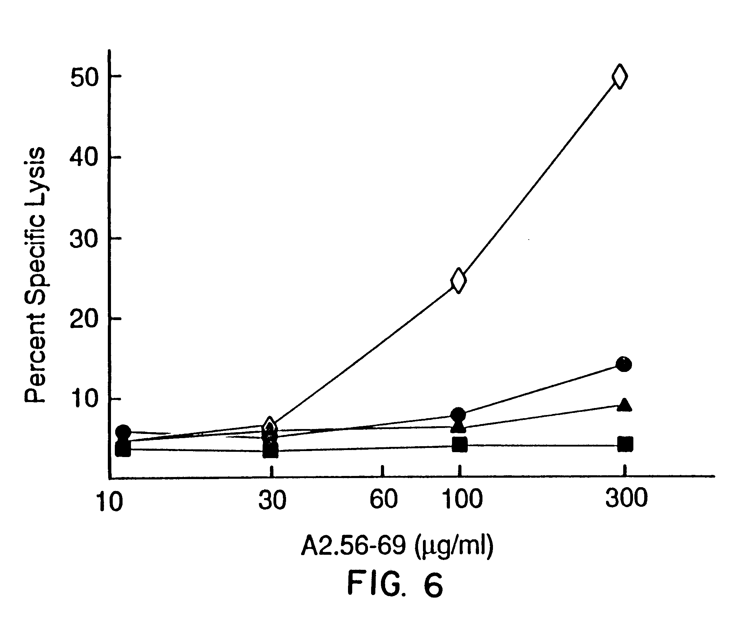

Clone A2/B17 was incubated with peptide A2.56-69 and $^{51}$Cr-labeled target cells at an effector-to-target ratio of 5:1 for 5 hours, after which $^{51}$chromium released was measured. The concentrations of peptide were 10, 30, 100, and 300 $\mu$g/ml. The results of the effect of peptide on the percent of specific lysis of the target cells by clone A2/B17 cells are presented in Fig. 6. The target cells were: (closed square), IBW4 (HLA-A3; B35; DR1); (closed triangle), LB (HLA-Aw68.1; B40, DR6); (closed circle), Pally (HLA-Aw68.2,26; B14,38; DR1,4), or (open diamond), IDF (HLA-Aw69,26; B18,38, DR5).

In the absence of peptide, clone A2/B17 cells do not lyse targets expressing HLA-Aw69, HLA-Aw68.1, and HLA-Aw68.2 (data not shown). The inability of clone A2/B17 cells to lyse these targets is due to the differences in the critical residues around position 62 from those found in HLA-A2 and HLA-B17. However, when peptide A2.56-69 was included in the cytotoxicity assay, there was significant lysis of HLA-Aw69 expressing targets by A2/B17 cells (Fig. 5). In contrast, targets expressing HLA-Aw68.1, HLA-Aw68.2, or the unrelated HLA-A3 molecule were not lysed.

Lysis of HLA-Aw69 cells by clone A2/B17 cells in the presence of peptide A2.56-69 was blocked by monoclonal antibody DR11-351, which only binds to the HLA-Aw69 of the target cell. In contrast, the monoclonal antibody MA2.1 did not inhibit lysis (results not shown). MA2.1 binds to the epitope of HLA-A2 and HLA-B17 formed by residues 56-69, but does not bind to the HLA-Aw69 or peptide A2.56-69. These results demonstrate the involvement of the HLA-Aw69 molecule in the sensitization by peptide A2.56-69.

The addition of A2.98-113 peptide to B cell lines which do not express HLA-A2 did not cause sensitization to lysis when target cells expressing a variety of HLA molecules were used. This was true even though a wide range of peptide concentrations (0.1 to 300 $\mu$g/ml were used.)

17

In binding A2.56-69, the HLA-Aw69 molecule is able to present an epitope that mimics the native structure of HLA-A2. That HLA-Aw69 but not other members of the HLA-A2/28 family can be sensitized is of interest. HLA-Aw69 is a recombinant molecule having $\alpha_1$ derived from HLA-Aw68 and $\alpha_2$ and $\alpha_3$ derived from HLA-A2.1 (Holmes and Parham, EMBO J. 4:2849 (1985)). Thus, HLA-2.1 and HLA-Aw69 differ by only 6 amino acids, all residing in the $\alpha_1$ domain and three of which are present in the A2.56-69 peptide.

Example 11

Locus of Peptide Interaction in Sensitization

To assess whether sensitization resulted from peptide interaction with the CTL or the target, cells were pretreated with A2.56-69, washed and then tested for cytolysis. More specifically, $1 \times 10^6$ clone A2/B17 cells or $^{51}$Cr-labeled IDF (HLA-Aw69,26; B18,38; DR5) were incubated with 100 $\mu$g of peptide or medium for 30 min. at 37°C, washed three times, and cytotoxicity as determined by $^{51}$chromium release was measured.

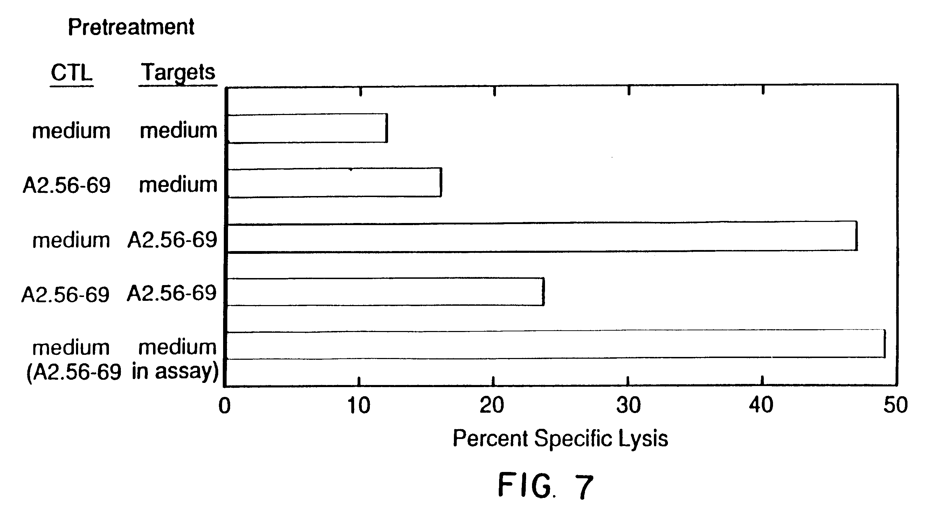

As seen from the results presented in Fig. 7, target cells expressing HLA-Aw69 were lysed when the targets, but not the CTL, were pretreated with A2.56-69.

Example 12

Effect of Peptide A2.56-69 on Release of Granules Containing Serine Esterase

The effect of peptide A2.56-69 on the release of granules containing serine esterase during co-culture of A2/B17 cells with HLA-Aw69 expressing cells may be essentially as described in Example 7, supra, except that the CTL are from clone A2/B17, the target cells are those expressing HLA-Aw69, and the cells are co-cultured in the absence or presence of peptide A2.56-69.

Although the foregoing invention has been described in some detail by way of illustration and example for purposes of clarity of understanding, it will be obvious that certain changes and modifications may be practiced within the scope of the appended claims.

UTILITY

It is evident from the above results that cytotoxic cells can be inhibited employing the subject peptides which are cross-reactive with the polymorphic regions of a Class I MHC antigen, as demonstrated with the A2 antigen. In this way, CTL's may be specifically inhibited from lysing target cells that express the same antigen that CTL's are restricted by. This is of use in the transplantation of organic tissue to prevent host rejection of tissue which is restricted by CTL. In addition, it may be of use in prevention/therapy of autoimmune disease.

Alternatively, the CTL's may be activated by employing specific conjugates involving the subject oligopeptides in conjunction with an antigen of interest. This is useful in activating CTL to lyse cells carrying antigens other than those recognized by the CTL, and thus may induce CTL to lyse cells carrying antigens which are cryptic to the host, for example, in parasitic diseases and in neoplasia.

The subject compounds may also be used in viral studies in determining MHC sequences associated with restriction of T-lymphocytes in the case of viral infection.

The peptides of the invention may also be used to determine the presence or absence of CTL which are targeted towards cells bearing the HLA-Class I molecule from which the peptide is derived. Knowledge of the presence or absence of the targeted CTL is useful in predicting the success or failure and/or requirement for CTL modulation in transplantation of organic tissue. It may also be helpful in determining the mechanisms whereby cells become cryptic to the immune system. In addition these peptides may also be helpful in the detection of autoimmune diseases.

**Claims**

1. A peptide linked to a functional moiety selected from a labelling group, an immunogen, a polypeptide antigen, a linker, and a cytotoxic agent;
    wherein the peptide modulates CTL (cytotoxic T-lymphocyte) activity and consists of a sequence of a least 8 amino acids coming within the extended sequence

18

$$aa^{55} \; G \; P \; E \; Y \; W \; D \; aa^{62} \; aa^{63} \; T \; aa^{65} \; aa^{66} \; aa^{67} \; K \; aa^{69} \; aa^{70} \; aa^{71}$$

$$Q \; T \quad aa^{74} \; R \; aa^{76} \; aa^{77} \; L \; aa^{79} \; aa^{80} \; aa^{81} \; aa^{82} \; aa^{83} \; Y \; Y \; N \; Q \; S \; E \; A \; G$$

$$S \; H \; aa^{94} \; aa^{95} \; Q \; aa^{97} \; M \; aa^{99} \; G \; C \; D \; aa^{103} \; G \; aa^{105} \; D \; aa^{107} \; R \; aa^{109} \; L \; R$$

$$G \; aa^{113} \; aa^{114} \; Q \; aa^{116} \; A \; Y \; D \; G$$

wherein:

$aa^{55}$ is E or K;

$aa^{62}$ is G, Q, E, or R;

$aa^{63}$ is an acidic amino acid or amide thereof;

$aa^{65}$ is Q, R or G;

$aa^{66}$ is I, N or K;

$aa^{67}$ is an aliphatic neutral amino acid;

$aa^{69}$ is an aliphatic neutral amino acid;

$aa^{70}$ is Q, H, S, N, or K;

$aa^{71}$ is an aliphatic neutral amino acid;

$aa^{74}$ is D, Y or H;

$aa^{76}$ is E or V;

$aa^{77}$ is D, S or N;

$aa^{79}$ is R or G;

$aa^{80}$ is T, I or N;

$aa^{81}$ is an aliphatic non-polar amino acid;

$aa^{82}$ is R or L;

$aa^{83}$ is G or R;

$aa^{94}$ is T or I;

$aa^{95}$ is a non-polar aliphatic amino acid of from 5 to 6 carbon atoms;

$aa^{97}$ is an aliphatic amino acid or W;

$aa^{99}$ is an aromatic amino acid;

$aa^{103}$ is a non-polar aliphatic amino acid of from 5 to 6 carbon atoms;

$aa^{105}$ is P or S;

$aa^{107}$ is G or W;

$aa^{109}$ is L or F;

$aa^{113}$ is Y or H;

$aa^{114}$ is H, Q, D, N, or R;

$aa^{116}$ is Y, D, S, F, or H;

with the proviso that the peptide is not a labelled peptide of the formula:

(i) R E T Q I C K A K

(ii) A Q T D R E $aa^{77}$ L R

where $aa^{77}$ is D, S or N;

(iii) G Y Y N Q S E A G S H T L Q $aa^{97}$

where $aa^{97}$ is S or R;

(iv) Y G C D V G P D G R.

2. A peptide according to claim 1 which consists of at least 8 amino acids coming within the sequence:

G S H T [V, I, or L] Q R M Y G C D V G S D [W or G] R
F L R G Y H Q Y A Y D G.

; or

Q E G P E Y W D (G or R) (E or N) T (R or Q) (K or N)
V K A (H or Q) S Q T (H or D) R (V or E) (D, S or N) L
(G or R) (T or I) (L or A) (R or L) (G or R) Y Y N Q S E A,

wherein the amino acids in the parentheses indicate that any of one amino acids in the parentheses is present.

3. A peptide as defined in claim 1 or 2 which is extended at either the N- or C-terminus by an amino acid sequence other than the wild type sequence of the protein from which said peptide is derived.

4. A pharmaceutical composition which comprises a peptide as defined in any one of the preceding claims, present in a pharmacologically effective dose in a pharmaceutically acceptable medium.

5. A pharmaceutical composition according to claim 4, wherein the peptide compound is selected from the sequences
T L Q R M Y G C D V G S D W R F L R G,
M Y G C D V G S D W R F L R G Y,
M Y G C D V G S D G R F L R G Y,
G P E Y W D G E T R K V K A, and
G P E Y W D R N T R N V K A.

6. An ex vivo method for determining the presence of MHC restricted cytotoxic T-lymphocytes (CTLs) which comprises;
contacting cytotoxic T-lymphocytes with a peptide as defined in any one of claims 1 to 3, said peptide covalently joined to a compound capable of providing a detectable signal; and
determining the presence of cells to which said detectable signal compound is specifically bound.

7. A peptide compound of at least 8 amino acids not more than about 30 amino acids, which peptide modulates CTL activity and consists of a sequence of at least 8 amino acids coming within the extended sequence defined in claim 1 for use in a method of treatment of the human or animal body.

8. A peptide for use according to claim 7 wherein the peptide is of the formula:
(A) as defined in claim 1 or 2;
(B)

G S H $aa^{94}$ $aa^{95}$ Q $aa^{97}$ M $aa^{99}$ G C D $aa^{103}$ G $aa^{105}$ D $aa^{107}$ R
$aa^{109}$ L R G $aa^{113}$ $aa^{114}$ Q $aa^{116}$ A Y D G;

wherein
$aa^{94}$ is T or I;
$aa^{95}$ is a non-polar aliphatic amino acid of from 5 to 6 carbon atoms;
$aa^{97}$ is an aliphatic amino acid or W;
$aa^{99}$ is an aromatic amino acid;
$aa^{103}$ is a non-polar aliphatic amino acid of from 5 to 6 carbon atoms;

20

aa$^{105}$ is P or S;

aa$^{107}$ is G or W;

aa$^{109}$ is L or F;

aa$^{113}$ is Y or H;

aa$^{114}$ is H, Q, D, N or R;

aa$^{116}$ is Y, D, S, F or H; or

(C)

$$aa^{55} \ G \ P \ E \ Y \ W \ D \ aa^{62} \ aa^{63} \ T \ aa^{65} \ aa^{66} \ aa^{67} \ K \ aa^{69} \ aa^{70} \ aa^{71}$$

$$Q \ T \ aa^{74} \ R \ aa^{76} \ aa^{77} \ aa^{79} \ aa^{80} \ aa^{81} \ aa^{82} \ aa^{83} \ Y \ Y \ N \ Q \ S \ E \ A$$

wherein

aa$^{55}$ is E or K, particularly E;

aa$^{62}$ is G, Q, E or R, particularly R or G;

aa$^{63}$ is an acidic amino acid or amide thereof; including E and N, particularly E;

aa$^{65}$ is Q, R or G, particularly Q or R;

aa$^{66}$ is I, N, or K, particularly N or K;

aa$^{67}$ is an aliphatic neutral amino acid including V, M, S, C and Y, particularly V;

aa$^{69}$ is an aliphatic neutral amino acid including, A, T and P, particularly A;

aa$^{70}$ is Q, H, S, N or K, particularly Q or H:

aa$^{71}$ is an aliphatic neutral amino acid including S, A and T, particularly S;

aa$^{74}$ is D, Y or H, particularly D or H;

aa$^{76}$ is E or V;

aa$^{77}$ is D, S or N, particularly D;

aa$^{79}$ is R or G, particularly G;

aa$^{80}$ is T, I or N, particularly T or I;

aa$^{81}$ is an aliphatic non-polar amino acid including L or A, particularly L;

aa$^{82}$ is R or L, particularly R;

aa$^{83}$ is G or R, particularly G;

for use in a method of treatment of the human or animal body.

9. A peptide for use according to claim 7 or 8 wherein the method of treatment comprises inhibiting the cytolytic activity of a cytotoxic T-lymphocyte.

10. A peptide for use according to claim 8 wherein the peptide is of the formula (C) and the method of treatment comprises sensitizing MHC restricted cells to a cytotoxic T-lymphocyte.

11. A peptide as defined in any one of claims 1 to 3 conjugated to an agent which causes cytotoxicity, for use in a method of inhibiting cytolytic activity of a CTL (Cytotoxic T-lymphocyte; by irreversibly binding the CTL.

**Patentansprüche**

1. Peptid, das an einen aus einer Markierungsgruppe, einem Immunogen einem Polypeptidantigen einem Linker und einem cytotoxischen Mittel gewählten funktionellen Teil gebunden ist,

worin das Peptid CTL (cytotoxische T-Lymphozyt)-Aktivität moduliert und aus einer Sequenz von mindestens 8 innerhalb folgender erweiterten Sequenz vorliegenden Aminosäuren besteht:

$$aa^{55} \ G \ P \ E \ Y \ W \ D \ aa^{62} \ aa^{63} \ T \ aa^{65} \ aa^{66} \ aa^{67} \ K \ aa^{69} \ aa^{70} \ aa^{71}$$

$$Q \ T \ aa^{74} \ R \ aa^{76} \ aa^{77} \ L \ aa^{79} \ aa^{80} \ aa^{81} \ aa^{82} \ aa^{83} \ Y \ Y \ N \ Q \ S \ E \ A \ G$$

$$S \ H \ aa^{94} \ aa^{95} \ Q \ aa^{97} \ M \ aa^{99} \ G \ C \ D \ aa^{103} \ G \ aa^{105} \ D \ aa^{107} \ R \ aa^{109} \ L \ R$$

$$G \ aa^{113} \ aa^{114} \ Q \ aa^{116} \ A \ Y \ D \ G$$

worin:

21

aa$^{55}$    E oder K ist;

aa$^{62}$    G, Q, E oder R ist;

aa$^{63}$    eine saure Aminosäure oder ein Amid davon ist;

aa$^{65}$    Q, R oder G ist;

aa$^{66}$    I, N oder K ist;

aa$^{67}$    eine aliphatische neutrale Aminosäure ist;

aa$^{69}$    eine aliphatische neutrale Aminosäure ist;

aa$^{70}$    Q, H, S, N oder K ist;

aa$^{71}$    eine aliphatische neutrale Aminosäure ist:

aa$^{74}$    D, Y oder H ist:

aa$^{76}$    E oder V ist;

aa$^{77}$    D, S oder N ist;

aa$^{79}$    R oder G ist;

aa$^{80}$    T, I oder N ist;

aa$^{81}$    eine aliphatische nicht-polare Aminosäure ist:

aa$^{82}$    R oder L ist;

aa$^{83}$    G oder R ist;

aa$^{94}$    T oder I ist;

aa$^{95}$    eine nicht-polare aliphatische Aminosäure mit 5 bis 6 Kohlenstofffatomen ist;

aa$^{97}$    eine aliphatische Aminosäure oder W ist;

aa$^{99}$    eine aromatische Aminosäure ist;

aa$^{103}$   eine nicht-polare aliphatische Aminosäure mit 5 bis 6 Kohlenstoffatomen ist;

aa$^{105}$   P oder S ist:

aa$^{107}$   G oder W ist;

aa$^{109}$   L oder F ist;

aa$^{113}$   Y oder H ist;

aa$^{114}$   H, Q, D, N oder R ist;

aa$^{116}$   Y, D, S, F oder H ist;

mit der Maßgabe, daß das Peptid nicht ein markiertes Peptid folgender Formel ist:

(i) R E T Q I C K A K

(ii) A Q T D R E aa$^{77}$ L R, worin aa$^{77}$ D, S oder N ist;

(iii) G Y Y N Q S E A G S H T L Q aa$^{97}$, worin aa$^{97}$ S oder R ist;

(iv) Y G C D V G P D G R.

2.    Peptid gemäß Anspruch 1, welches aus mindestens 8 innerhalb folgender erweiterten Sequenz vorliegenden Aminosäuren besteht:

G S H T [V, I oder L] Q R M Y G C D V G S D [W oder G] R F L R G Y H Q
Y A Y D G;

oder

Q E G P E Y W D (G oder R) (E oder N) T (R oder Q) (K oder N) V K A
(H  oder Q) S Q T (H oder D) R (V oder E) (D, S oder N) L (G oder R)
(T oder I)  (L oder A) (R oder L) (G oder R) Y Y N Q S E A.

worin die Aminosäuren in den Klammern anzeigen, daß eine beliebige der Aminosäuren in den Klammern vorliegt.

3.    Peptid nach Anspruch 1 oder 2, welches entweder an dem N- oder C-Terminus durch eine Aminosäuresequenz erweitert ist, die eine andere als die Sequenz vom Wild-Typ des Proteins ist, von dem das Peptid abgeleitet ist.

4.  Pharmazeutische Zusammensetzung, welche ein Peptid enthält, wie es in mindestens einem der vorhergehenden Ansprüche definiert ist, vorliegend in einer pharmakologisch wirksamen Dosis in einem pharmazeutisch zulässigen Medium.

5.  Pharmazeutische Zusammensetzung gemäß Anspruch 4, worin die Peptidverbindung aus folgenden Sequenzen gewählt ist.

    T L Q R M Y G C D V G S D W R F L R G,
    M Y G C D V G S D W R F L R G Y,
    M Y G C D V G S D G R F L R G Y,
    G P E Y W D G E T R K V K A und
    G P E Y W D R N T R N V K A.

6.  <u>Ex vivo</u>-Verfahren zur Bestimmung der Anwesenheit von MHC-restringierten cytotoxischen T-Lymphozyten (CTLs), welches folgendes umfaßt:

    Kontaktieren cytotoxischer T-Lymphozyten mit einem Peptid, wie es nach mindestens einem der Ansprüche 1 bis 3 definiert ist, wobei das Peptid kovalent an eine Verbindung gebunden ist, die zur Bereitstellung eines detektierbaren Signals in der Lage ist; und

    Bestimmen der Anwesenheit von Zellen, an denen die ein detektierbares Signal hervorbringende Verbindung spezifisch gebunden ist.

7.  Peptidverbindung mit mindestens 8 Aminosäuren und nicht mehr als etwa 30 Aminosäuren, wobei das Peptid CTL-Aktivität moduliert und aus einer Sequenz von mindestens 8 innerhalb der erweiterten Sequenz vorliegenden Aminosäuren, definiert in Anspruch 1, besteht, zur Verwendung bei einem Verfahren zur Behandlung des menschlichen oder tierischen Körpers.

8.  Peptid zur Verwendung gemäß Anspruch 7, worin das Peptid folgende Formel aufweist:

    (A) wie in Anspruch 1 oder 2 definiert;

    (B)

$$G\ S\ H\ aa^{94}\ aa^{95}\ Q\ aa^{97}\ M\ aa^{99}\ G\ C\ D\ aa^{103}\ G\ aa^{105}\ D\ aa^{107}\ R\ aa^{109}$$
$$L\ R\ G\ aa^{113}\ aa^{114}\ Q\ aa^{116}\ A\ Y\ D\ G;$$

worin

| | |
|---|---|
| $aa^{94}$ | T oder I ist; |
| $aa^{95}$ | eine nicht-polare aliphatische Aminosäure mit 5 bis 6 Kohlenstoffatomen ist; |
| $aa^{97}$ | eine aliphatische Aminosäure oder W ist; |
| $aa^{99}$ | eine aromatische Aminosäure ist; |
| $aa^{103}$ | eine nicht-polare aliphatische Aminosäure mit 5 bis 6 Kohlenstoffatomen ist; |
| $aa^{105}$ | P oder S ist; |
| $aa^{107}$ | G oder W ist; |
| $aa^{109}$ | L oder F ist; |
| $aa^{113}$ | Y oder H ist; |
| $aa^{114}$ | H, Q, D, N oder R ist; |
| $aa^{116}$ | Y, D, S, F oder H ist; oder |

(C)

$$aa^{55}\ G\ P\ E\ Y\ W\ D\ aa^{62}\ aa^{63}\ T\ aa^{65}\ aa^{66}\ aa^{67}\ K\ aa^{69}\ aa^{70}\ aa^{71}\ Q\ T\ aa^{74}\ R$$
$$aa^{76}\ aa^{77}\ aa^{79}\ aa^{80}\ aa^{81}\ aa^{82}\ aa^{83}\ Y\ Y\ N\ Q\ S\ E\ A$$

worin

| | |
|---|---|
| $aa^{55}$ | E oder K, insbesondere E ist; |
| $aa^{62}$ | G, Q, E oder R, insbesondere R oder G ist; |
| $aa^{63}$ | eine saure Aminosäure oder ein Amid davon ist; einschließend E und N, insbesondere E; |
| $aa^{65}$ | Q, R oder G, insbesondere Q oder R ist; |

aa$^{66}$    I, N oder K, insbesondere N oder K ist;

aa$^{67}$    eine aliphatische neutrale Aminosäure ist, einschließend V, M, S, C und Y, insbesondere V;

aa$^{69}$    eine aliphatische neutrale Aminosäure ist, einschließend A, T und P, insbesondere A;

aa$^{70}$    Q, H, S, N oder K, insbesondere Q oder H ist;

aa$^{71}$    eine aliphatische neutrale Aminosäure ist, einschließend S, A und T, insbesondere S;

aa$^{74}$    D, Y oder H, insbesondere D oder H ist;

aa$^{76}$    E oder V ist;

aa$^{77}$    D, S oder N, insbesondere D ist;

aa$^{79}$    R oder G, insbesondere G ist;

aa$^{80}$    T, I oder N, insbesondere T oder I ist;

aa$^{81}$    eine aliphatische nicht-polare Aminosäure ist, einschließend L oder A, insbesondere L;

aa$^{82}$    R oder L, insbesondere R ist;

aa$^{83}$    G oder R, insbesondere G ist;

zur Verwendung bei einem Verfahren der Behandlung des menschlichen oder tierischen Körpers.

**9.** Peptid zur Verwendung gemäß Anspruch 7 oder 8, worin das Verfahren der Behandlung die Inhibierung der cytolytischen Aktivität eines cytotoxischen T-Lymphozyten inhibiert.

**10.** Peptid zur Verwendung nach Anspruch 8, worin das Peptid die Formel (C) besitzt und das Verfahren der Behandlung das Sensibilisieren von MHC-restringierten Zellen auf einen cytotoxischen T-Lymphozyten umfaßt.

**11.** Peptid, wie es nach mindestens einem der Ansprüche 1 bis 3 definiert ist, konjugiert an einem Mittel, welches Cytotoxizität verursacht, zur Verwendung bei einem Vefahren der Inhibierung cytolytischer Aktivität eines CTL (cytotoxischer T-Lymphozyt) durch irreversible Bindung des CTL.

## Revendications

**1.** Peptide lié à une partie fonctionnelle choisie parmi un groupe de marquage, un immunogène, un antigène polypeptidique, un linker, et un agent cytotoxique ;

dans lequel le peptide module l'activité des CTL (lymphocytes-T cytotoxiques) et consiste en une séquence d'au moins 8 acides aminés intervenant dans la séquence prolongée

$$aa^{55}\ G\ P\ E\ Y\ W\ D\ aa^{62}\ aa^{63}\ T\ aa^{65}\ aa^{66}\ aa^{67}\ K\ aa^{69}\ aa^{70}\ aa^{71}\ Q\ T$$
$$aa^{74}\ R\ aa^{76}\ aa^{77}\ L\ aa^{79}\ aa^{80}\ aa^{81}\ aa^{82}\ aa^{83}\ Y\ Y\ N\ Q\ S\ E\ A\ G\ S\ H\ aa^{94}\ aa^{95}\ Q$$
$$aa^{97}\ M\ aa^{99}\ G\ C\ D\ aa^{103}\ G\ aa^{105}\ D\ aa^{107}\ R\ aa^{109}\ L\ R\ G\ aa^{113}\ aa^{114}\ Q\ aa^{116}\ A\ Y$$
$$D\ G$$

dans laquelle :

aa$^{55}$ est E ou K ;

aa$^{62}$ est G, Q, E ou R ;

aa$^{63}$ est un acide aminé acide ou un amide de celui-ci ;

aa$^{65}$ est Q, R ou G ;

aa$^{66}$ est I, N ou K ;

aa$^{67}$ est un acide aminé neutre aliphatique ;

aa$^{69}$ est un acide aminé neutre aliphatique ;

aa$^{70}$ est Q, H, S, N ou K ;

aa$^{71}$ est un acide aminé neutre aliphatique ;

aa$^{74}$ est D, Y ou H ;

aa$^{76}$ est E ou V ;

aa$^{77}$ est D, S ou N ;

aa$^{79}$ est R ou G ;

aa$^{80}$ est T, I ou N ;

aa$^{81}$ est un acide aminé non polaire aliphatique ;

aa$^{82}$ est R ou L ;

aa$^{83}$ est G ou R ;

aa$^{94}$ est T ou I ;

aa$^{95}$ est un acide aminé aliphatique non polaire de 5 à 6 atomes de carbone ;

aa$^{97}$ est un acide aminé aliphatique ou W ;

aa$^{99}$ est un acide aminé aromatique ;

aa$^{103}$ est un acide aminé aliphatique non polaire de 5 à 6 atomes de carbone ;

aa$^{105}$ est P ou S ;

aa$^{107}$ est G ou W ;

aa$^{109}$ est L ou F ;

aa$^{113}$ est Y ou H ;

aa$^{114}$ est H, Q, D, N ou R ;

aa$^{116}$ est Y, D, S, F ou H ;

à la condition que le peptide ne soit pas un peptide marqué de formule :

(i) R E T Q I C K A K

(ii) A Q T D R E aa$^{77}$ L R

où aa$^{77}$ est D, S, ou N ;

(iii) G Y Y N Q S E A G S H T L Q aa$^{97}$

où aa$^{97}$ est S ou R ;

(iv) Y G C D V G P D G R.

2. Peptide selon la revendication 1 qui consiste en au moins 8 acides aminés présents dans la séquence :

G S H T [V, I, ou L] Q R M Y G C D V G S D [W ou G] R F L R G Y H
Q Y A Y D G.

; ou

Q E G P E Y W D (G ou R) (E ou N) T (R ou Q) (K ou N) V K A (H ou
Q) S Q T (H ou D) R (V ou E) (D, S ou N) L (G ou R) (T ou I) (L ou A) (R ou L) (G ou
R) Y Y N Q S E A,

dans laquelle les acides aminés entre parenthèses indiquent que l'un quelconque des acides aminés entre parenthèses est présent.

3. Peptide tel que défini à la revendication 1 ou 2 qui est prolongé soit à l'extrémité N-terminale soit à l'extrémité C-terminale par une séquence d'acides aminés autre que la séquence de type sauvage de la protéine dont est dérivé ledit peptide.

4. Composition pharmaceutique qui comprend un peptide tel que défini dans l'une quelconque des revendications précédentes, présent à une dose pharmacologiquement efficace dans un milieu pharmaceutiquement acceptable.

5. Composition pharmaceutique selon la revendication 4, dans laquelle le composé peptidique est choisi parmi les séquences

T L Q R M Y G C D V G S D W R F L R G,

M Y G C D V G S D W R F L R G Y,

M Y G C D V G S D G R F L R G Y,

G P E Y W D G E T R K V K A, et

G P E Y W D R N T R N V K A.

**6.** Méthode <u>ex vivo</u> pour déterminer la présence de lymphocytes-T cytotoxiques (CTL) restreints au CMH qui comprend les étapes consistant :

à mettre en contact des lymphocytes-T cytotoxiques avec un peptide tel que défini dans l'une quelconque des revendications 1 à 3, ledit peptide étant lié par liaison covalente à un composé capable de fournir un signal détectable ; et

à déterminer la présence de cellules auxquelles ledit composé avec un signal détectable est lié de manière spécifique.

**7.** Composé peptidique d'au moins 8 acides aminés et pas plus d'environ 30 acides aminés, peptide qui module l'activité desCTL et consiste en une séquence d'au moins 8 acides aminés présents dans la séquence prolongée définie à la revendication 1 pour utilisation dans une méthode de traitement du corps humain ou animal.

**8.** Peptide pour utilisation selon la revendication 7 dans lequel le peptide est de la formule :

(A) telle que définie dans la revendication 1 ou 2 ;

(B)

$$G\ S\ H\ aa^{94}\ aa^{95}\ Q\ aa^{97}\ M\ aa^{99}\ G\ C\ D\ aa^{103}\ G\ aa^{105}\ D\ aa^{107}$$

$$R\ aa^{109}\ L\ R\ G\ aa^{113}\ aa^{114}\ Q\ aa^{116}\ A\ Y\ D\ G\ ;$$

dans laquelle

$aa^{94}$ est T ou I ;

$aa^{95}$ est un acide aminé aliphatique non polaire de 5 à 6 atomes de carbone ;

$aa^{97}$ est un acide aminé aliphatique ou W ;

$aa^{99}$ est un acide aminé aromatique ;

$aa^{103}$ est un acide aminé aliphatique non polaire de 5 à 6 atomes de carbone ;

$aa^{105}$ est P ou S ;

$aa^{107}$ est G ou W ;

$aa^{109}$ est L ou F ;

$aa^{113}$ est Y ou H ;

$aa^{114}$ est H, Q, D, N ou R ;

$aa^{116}$ est Y, D, S, F ou H ; ou

(C)

$$aa^{55}\ G\ P\ E\ Y\ W\ D\ aa^{62}\ aa^{63}\ T\ aa^{65}\ aa^{66}\ aa^{67}\ K\ aa^{69}\ aa^{70}\ aa^{71}\ Q$$

$$T\ aa^{74}\ R\ aa^{76}\ aa^{77}\ aa^{79}\ aa^{80}\ aa^{81}\ aa^{82}\ aa^{83}\ Y\ Y\ N\ Q\ S\ E\ A$$

dans laquelle

$aa^{55}$ est E ou K, en particulier E ;

$aa^{62}$ est G, Q, E ou R, en particulier R ou G ;

$aa^{63}$ est un acide aminé acide ou un amide de celui-ci ; y compris E et N, en particulier E ;

$aa^{65}$ est Q, R ou G, en particulier Q ou R ;

$aa^{66}$ est I, N ou K, en particulier N ou K ;

$aa^{67}$ est un acide aminé neutre aliphatique comprenant V, M, S, C et Y, en particulier V ;

$aa^{69}$ est un acide aminé neutre aliphatique comprenant A, T et P, en particulier A ;

$aa^{70}$ est Q, H, S, N ou K, en particulier Q ou H ;

$aa^{71}$ est un acide aminé neutre aliphatique comprenant S, A et T, en particulier S ;

$aa^{74}$ est D, Y ou H, en particulier D ou H ;

$aa^{76}$ est E ou V ;

$aa^{77}$ est D, S ou N, en particulier D ;

$aa^{79}$ est R ou G, en particulier G ;

$aa^{80}$ est T, I ou N, en particulier T ou I ;

$aa^{81}$ est un acide aminé non polaire aliphatique comprenant L ou A, en particulier L ;

26

aa$^{82}$ est R ou L, en particulier R ;

aa$^{83}$ est G ou R, en particulier G ;

pour utilisation dans une méthode de traitement du corps humain ou animal.

9. Peptide pour utilisation selon la revendication 7 ou 8 où la méthode de traitement comprend l'inhibition de l'activité cytolytique d'un lymphocyte-T cytotoxique.

10. Peptide pour utilisation selon la revendication 8 où le peptide est de formule (C) et la méthode de traitement comprend une étape consistant à sensibiliser à un lymphocyte-T cytotoxique des cellules à restriction au CMH ;

11. Peptide tel que défini dans l'une quelconque des revendications 1 à 3 conjugué à un agent qui cause la cytotoxicité, pour utilisation dans une méthode d'inhibition de l'activité cytolytique d'un CTL (lymphocyte-T cytotoxique ; par liaison irréversible au CTL.

|  |  | Sequence | % Inhibition |
|---|---|---|---|
| A2 | .98-113 | M Y G C D V G S D W R F L R G Y | 74 |
| Aw 68 | .98-113 | ├──────────────G──────────────┤ | 0 |
| A2 | .98-108 | ├──────────────────────┤ | 60 |
| A2 | .99-108 | ├─────────────────────┤ | 36 |
| A2 | 100-108 | ├────────────────────┤ | 60 |
| A2 | 101-108 | ├──────────────────┤ | 55 |
| A2 | 102-108 | ├────────────────┤ | 15 |
| A2 | 103-108 | ├──────────────┤ | 13 |
| A2 | 104-108 | ├────────────┤ | 0 |

## FIG. 1

FIG. 2

FIG. 3B

CTL/Target Ratio

Percent Specific Esterase Release

FIG. 3A

CTL/Target Ratio

Percent Specific Esterase Release

**FIG. 4**

α1

A2/B17

```
           1        10        20        30        40        50        60|       70        80        90
consensus  GSHSMRYFYTSVSRPGRGEPRFIAVGYVDDTQFVRFDSDAASPRMEPRAPWIEQEGPEYWDRETQIVKAQSQTDREDLRTLRGYYNQSEA
B17(Bw58)  ----------AM---------------------------------------T-----------G--RNM--SA--Y--N--IALR-------
A2.1       ---------F-----------------------------------Q----------G--RK---H---H-V--G-----------
A2.3       ---------F-----------------------------------Q----------G--RK---H---H-V--G-----------
A2.2F      ---------F-----------------------------------R----------G--RK---H---H-V--G-----------
Aw69       ----------------------------------------------Q-------------N-RN---------V--G---------
Aw68.2     ----------M----------------------------------Q-------------N-RN---------V--G---------
Aw68.1     ----------------------------------------------Q------------N-RN------V--G---------
A3.1       ---------F----------------------------- -----Q----------Q--RN---------V--G---------
consensus  GSHSMRYFYTSVSRPGRGEPRFIAVGYVDDTQFVRFDSDAASPRMEPRAPWIEQEGPEYWDRETQIVKAQSQTDREDLRTLRGYYNQSEA
```

α2

A2/Aw69

```
           100|      110       120       130       140       150       160       170       180
consensus  GSHTIQRMYGCDVGPDGRLLRGYHQYAYDGKDYIALNEDLRSWTAADTAAQITQRKWEAARVAEQLRAYLEGTCVEWLRRYLENGKETLQRA
B17(Bw58)  ----I-------L--------HD-S-----------S-----------------------L------------
A2.1       ----V---------SSWGF-----------------K-----------M---T-KH----H------------------T
A2.3       ----V---------S-W-F-----------------K-----------M---T-KH---T-HE---W-----------T
A2.2F      --L-----------S+W+F-----------------K-----------M---T-KH---------W-----------T
Aw69       ----V---------S-W-F-----------------K-----------M---T-KH----H------------------T
Aw68.2     --------------F---------------------K-----------M---T-KH----H----W-----------T
Aw68.1     ------M-------S-F-----R-D-----------K-----------M---T-KH----H----W-----------T
A3.1       ------I-------S---F---R-D-----------M-----K-----HE---------D---------------T
consensus  GSHTIQRMYGCDVGPDGRLLRGYHQYAYDGKDYIALNEDLRSWTAADTAAQITQRKWEAARVAEQLRAYLEGTCVEWLRRYLENGKETLQRA
```

α3

```
           190       200       210       220       230       240       250       260       270
consensus  DPPKTHVTHHPISDHEATLRCWALGFYPAEITLTWQRDGEDQTQDTELVETRPAGDGTFQKWAAVVVPSGEEQRYTCHVQHEGLPKPLTLRW
B17(Bw58)  --------------V----------------------------------R-------------------
A2.1       -A----M--AV-------------S--------------------------Q-----------
A2.3       -A----M--AV-------------S--------------------------Q-----------
A2.2F      -A----M--AV-------------S--------------------------Q-----------
Aw69       -A----M--AV-------------S--------------------------Q-----------
Aw68.2     -A----M--AV-------------S-------------------V------Q-----------
Aw68.1     -A----M--AV-------------S-------------------V------Q-----------
A3.1       ------M-----------------------------------------------------
consensus  DPPKTHVTHHPISDHEATLRCWALGFYPAEITLTWQRDGEDQTQDTELVETRPAGDGTFQKWAAVVVPSGEEQRYTCHVQHEGLPKPLTLRW
```

FIG. 5A

FIG. 5B

FIG. 5C

FIG. 5D

FIG. 6

EP 0 365 525 B1

Pretreatment

CTL        Targets

medium     medium

A2.56-69   medium

medium     A2.56-69

A2.56-69   A2.56-69

medium     medium
(A2.56-69 in assay)

0      10     20     30     40     50

Percent Specific Lysis

FIG. 7

EP 0 365 525 B1